(19) Europäisches Patentamt
European Patent Office
Office européen des brevets

(11) **EP 4 371 410 A1**

(12) **EUROPEAN PATENT APPLICATION**
published in accordance with Art. 153(4) EPC

(43) Date of publication:
**22.05.2024 Bulletin 2024/21**

(21) Application number: **22842333.1**

(22) Date of filing: **29.06.2022**

(51) International Patent Classification (IPC):
***A01N 33/12*** *(2006.01)*

(52) Cooperative Patent Classification (CPC):
(C-Sets available)
**A01P 1/00; A01N 33/12; C12Q 1/18;** Y02A 50/30

(Cont.)

(86) International application number:
**PCT/KR2022/009358**

(87) International publication number:
**WO 2023/287071 (19.01.2023 Gazette 2023/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.07.2021 KR 20210093569**

(71) Applicant: **LG Chem, Ltd.**
**Seoul 07336 (KR)**

(72) Inventors:
- **LEE, Ji Seok**
  **Daejeon 34122 (KR)**
- **KANG, Soonhee**
  **Daejeon 34122 (KR)**
- **KIM, Sanggon**
  **Daejeon 34122 (KR)**
- **BAEK, Leehyeon**
  **Daejeon 34122 (KR)**
- **YUN, Haesung**
  **Daejeon 34122 (KR)**
- **LEE, Hwanhee**
  **Daejeon 34122 (KR)**
- **CHUNG, Da Sol**
  **Daejeon 34122 (KR)**
- **JUNG, Seonjung**
  **Daejeon 34122 (KR)**
- **CHOI, Hyungsam**
  **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

# (54) ANTIBACTERIAL COMPOSITION

(57) The present specification relates to an antibacterial composition comprising at least one compound comprising a quaternary ammonium structure having an acrylate group or methacrylate group, and having an antibacterial strength A of 90% or more which are measured by the following Method 1 for at least one strain of Gram-positive bacteria and Gram-negative bacteria and an acute oral toxicity dose LD50 of 300 mg/Kg or more.

[Figure 1]

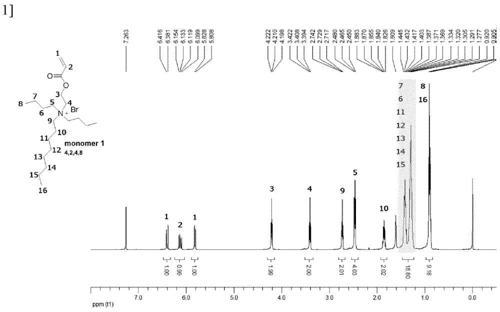


EP 4 371 410 A1

(52) Cooperative Patent Classification (CPC): (Cont.)

C-Sets
**A01N 33/12, A01N 25/10**

## Description

[Technical Field]

This application claims priority to and the benefit of Korean Patent Application No. 10-2021-0093569 filed in the Korean Intellectual Property Office on July 16, 2021, the entire contents of which are incorporated herein by reference.
The present invention relates to an antibacterial composition.

[Background Art]

Recently, various products such as daily supplies or hygiene products are required to have antibacterial properties.
The degree of antibacterial properties required and the material requirements for imparting antibacterial properties differ depending on the material of the product requiring antibacterial properties and the state of final use. For example, the properties of the material for imparting antibacterial properties and the degree of antibacterial properties vary depending on the amount of antibacterial material applied to a product and the materials used together.
Therefore, there is a need for developing an antibacterial material suitable for application to each of various products.

[Detailed Description of the Invention]

[Technical Problem]

The present invention has been made in an effort to provide an antibacterial composition comprising an antibacterial material which has hydrophilicity and hydrophobicity, and thus, is advantageous for imparting antibacterial properties, and is capable of not only imparting antibacterial properties but also solving safety problems due to the leakage of antibacterial materials, by forming a copolymer with an acryl-based resin and the like.

[Technical Solution]

An exemplary embodiment of the present invention provides an antibacterial composition comprising at least one compound comprising a quaternary ammonium structure having an acrylate group or methacrylate group, and having an antibacterial strength A of 90% or more which is measured by the following Method 1 for at least one strain of Gram-positive bacteria and Gram-negative bacteria and an acute oral toxicity dose LD50 of 300 mg/Kg or more:

[Method 1]

After 25 ml of a broth-type medium (Nutrient broth, BD DIFCO., 8 g/L) inoculated with 3,000 CFU/ml bacteria is put into a 50 mL conical tube, 0.015 g of the antibacterial composition is added thereto and suspended (vortexing), a sufficiently mixed solution is incubated for 16 hours in a shaking water bath maintained at 35°C.
After the incubated solution was diluted to 1/5 using a 1X PBS buffer solution, absorbance (λ = 600 nm) was measured using a UV/Vis spectrophotometer, and the antibacterial strength, which is a bacteriostatic reduction rate, is calculated by the following equation by comparing the measured absorbance with a solution incubated without the addition of the antibacterial composition.

$$\text{Antibacterial strength (\%)} = (1 - A_{sample}/A_{Reference}) \times 100$$

$A_{sample}$ = Absorbance of medium solution incubated by adding antibacterial composition

$A_{reference}$ = Absorbance of medium solution incubated without addition of antibacterial composition

According to another exemplary embodiment of the present invention, Method 1 is measured against at least one strain of *Proteus mirabilis, E.coli, S.aureus*, *E.Cloaceae* and

*E.faecalis.*

According to still another exemplary embodiment of the present invention, Method 1 is measured for each strain of *Proteus mirabilis, E.coli, S.aureus*, *E.Cloaceae* and *E.faecalis.*

According to yet another exemplary embodiment of the present invention, the antibacterial strength A of the antibacterial composition is expressed within 1 hour.

According to yet another exemplary embodiment of the present invention, the ratio (CB) of the antibacterial strength C measured by the following Method 3 to the antibacterial strength B measured by the following Method 2 of the antibacterial composition is 1 or more and less than 2:

[Method 2]

This method is the same as Method 1, except that 0.005 g of the antibacterial composition is added instead of 0.015 g of the antibacterial composition.

[Method 3]

This method is the same as Method 1, except that 0.02 g of the antibacterial composition is added instead of 0.015 g of the antibacterial composition.

According to yet another exemplary embodiment of the present invention, provided is a product comprising the antibacterial composition or prepared therefrom.

According to yet another exemplary embodiment of the present invention, provided is a compound selected among the following Monomers 1 to 10.

Monomer 1

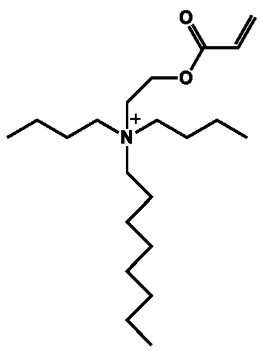

Monomer 2

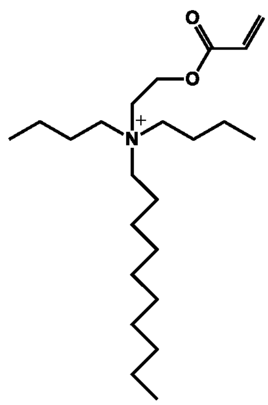

Monomer 3

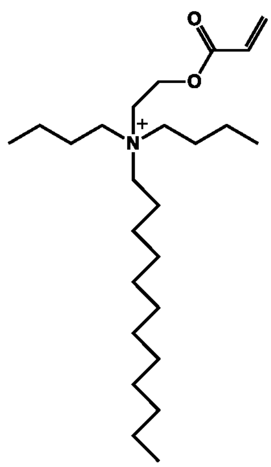

Monomer 4

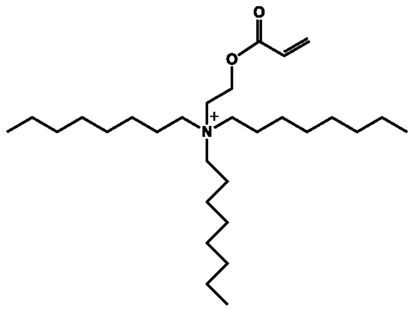

Monomer 5

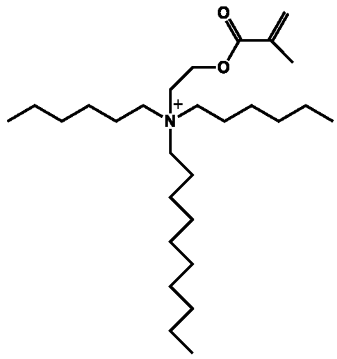

Monomer 6

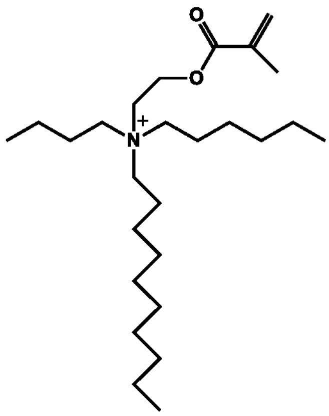

Monomer 7

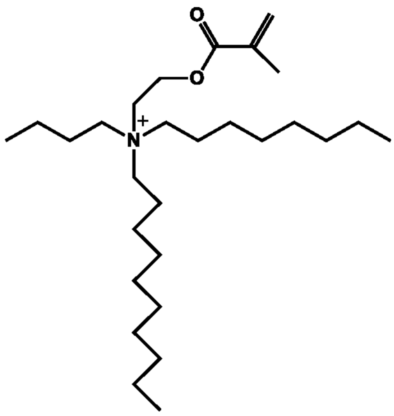

Monomer 8

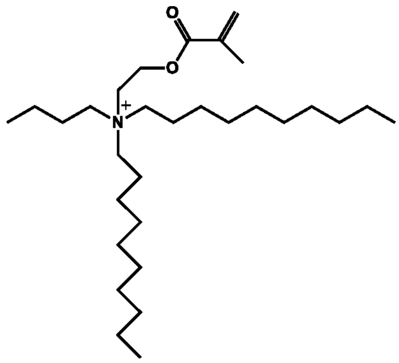

Monomer 9

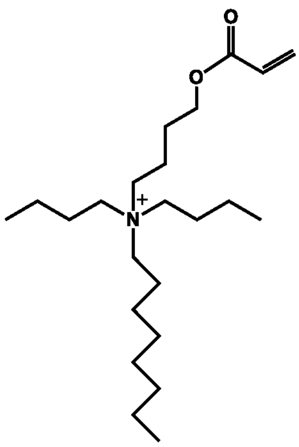

Monomer 10

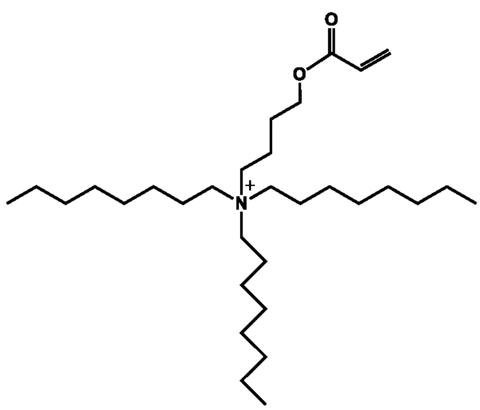

[Advantageous Effects]

The antibacterial composition according to some exemplary embodiments of the present invention is an antibacterial composition comprising an antibacterial material which has hydrophilicity and hydrophobicity, and thus, is

advantageous for imparting antibacterial properties, and is capable of not only imparting antibacterial properties but also solving safety problems due to the leakage of antibacterial materials, by forming a copolymer with an acryl-based resin, has antibacterial properties and acute oral toxicity dose controlled within a characteristic range, and is useful as a material capable of safely imparting excellent antibacterial properties.

The antibacterial composition according to some exemplary embodiments of the present invention can exhibit antibacterial properties within a short period of time.

Since the antibacterial composition according to some exemplary embodiments of the present invention has little change in antibacterial strength depending on the amount of antibacterial material used, antibacterial properties can be exhibited within an expected range even when the unevenness of concentration occurs unintentionally during application to a product.

[Brief Description of Drawings]

FIG. 1 is a view illustrating the $^1$H-NMR spectrum ($(CD_3)_2SO$) of synthesized Monomer 1 as an exemplary embodiment of the present invention.

FIG. 2 is a view illustrating the $^1$H-NMR spectrum ($(CD_3)_2SO$) of synthesized Monomer 2 as an exemplary embodiment of the present invention.

FIG. 3 is a view illustrating the $^1$H-NMR spectrum ($(CD_3)_2SO$) of synthesized Monomer 3 as an exemplary embodiment of the present invention.

[Best Mode]

An exemplary embodiment of the present invention provides an antibacterial composition comprising at least one compound comprising a quaternary ammonium structure having an acrylate group or a methacrylate group, and having an antibacterial strength A of 90% or more which is measured by the following Method 1 for at least one strain of Gram-positive bacteria and Gram-negative bacteria and an acute oral toxicity dose LD50 of 300 mg/Kg or more:

[Method 1]

After 25 ml of a broth-type medium (Nutrient broth, BD DIFCO., 8 g/L) inoculated with 3,000 CFU/ml bacteria is put into a 50 mL conical tube, 0.015 g of the antibacterial composition is added thereto and suspended (vortexing), a sufficiently mixed solution is incubated for 16 hours in a shaking water bath maintained at 35°C.

After the incubated solution was diluted to 1/5 using a 1X PBS buffer solution, absorbance ($\lambda$ = 600 nm) was measured using a UV/Vis spectrophotometer, and the antibacterial strength, which is a bacteriostatic reduction rate, is calculated by the following equation by comparing the measured absorbance with a solution incubated without the addition of the antibacterial composition.

$$\text{Antibacterial strength (\%)} = \left(1 - {A_{sample}}/{A_{Reference}}\right) \times 100$$

$A_{sample}$ = Absorbance of medium solution incubated by adding antibacterial composition

$A_{reference}$ = Absorbance of medium solution incubated without addition of antibacterial composition

A compound comprising a quaternary ammonium structure having an acrylate group or methacrylate group comprised in the antibacterial composition may physicochemically destruct and kill a cell surface layer structure by hydrophobic interaction, when cations of the ammonium molecule of the quaternary ammonium-based compound are electrostatically adsorbed onto anionic sites of the cell surface of bacteria or microorganisms and a substituent bound to the quaternary ammonium structure is hydrophobic. Further, an antibacterial function may be introduced through copolymerization with an acryl-based resin and the like by the acrylate group or methacrylate group, and in this case,

safety may be improved by preventing the antibacterial material from being leaked. According to the exemplary embodiments of the present invention, high antibacterial properties may be safely imparted by comprising compounds having the quaternary ammonium structure as described above and simultaneously limiting the antibacterial strength and acute oral toxicity dose of the antibacterial composition to within a specific range.

The antibacterial strength mentioned in the present specification may be measured for at least one strain of *Proteus mirabilis, E.coli, S.aureus*, *E.Cloaceae* and *E.faecalis.* Preferably, the antibacterial strength mentioned in the present specification may be measured for each of *Proteus mirabilis, E.coli, S.aureus*, *E.Cloaceae* and *E.faecalis.* The antibacterial composition according to exemplary embodiments of the present invention may have a certain level or more of antibacterial strength against Gram-positive bacteria and Gram-negative bacteria. ATCC29906 and CCUG4637 may be used as *Proteus mirabilis,* ATCC13047, ATCC13048, and CCUG71839 may be used as *E.Cloaceae*, and ATCC29212 and CCUG9997 may be used as *E.faecalis.*

According to another exemplary embodiment of the present invention, the antibacterial strength A of the antibacterial composition is expressed within 1 hour. This enables a desired antibacterial strength to be exhibited immediately after application of the antibacterial composition to an intended use or product.

According to still another exemplary embodiment of the present invention, the ratio (CB) of the antibacterial strength C measured by the following Method 3 to the antibacterial strength B measured by the following Method 2 of the antibacterial composition is 1 or more and less than 2:

[Method 2]

This method is the same as Method 1, except that 0.005 g of the antibacterial composition is added instead of 0.015 g of the antibacterial composition.

[Method 3]

This method is the same as Method 1, except that 0.02 g of the antibacterial composition is added instead of 0.015 g of the antibacterial composition.

When the antibacterial composition is applied to the intended use or product, regions of unintentionally high concentration and low concentration of the antibacterial material may occur, and a region of low concentration may not exhibit the desired antibacterial properties. However, according to the exemplary embodiments, since there is little change in antibacterial strength depending on the amount of antibacterial material used, antibacterial properties can be exhibited within an expected range even when the unevenness of concentration occurs unintentionally during application to an intended use or product.

According to yet another exemplary embodiment of the present invention, the antibacterial strength A is 99% or more, preferably 99.3% or more, more preferably 99.5% or more, even more preferably 99.7% or more, and still even more preferably 99.9% or more.

According to yet another exemplary embodiment of the present invention, an antibacterial strength D measured by the following Method 4 is 65% or more:

[Method 4]

This method is the same as Method 1, except that 0.01 g of the antibacterial composition is added instead of 0.015 g of the antibacterial composition.

According to the exemplary embodiments, excellent antibacterial properties may be provided even when the antibacterial composition is added in an amount of 0.01 g.

According to another exemplary embodiment of the present invention, the antibacterial strength D may be 65% or more, 68% or more, 68.2% or more, and 70% or more. Further, the antibacterial strength D may be 99% or more, preferably 99.3% or more, more preferably 99.5% or more, even more preferably 99.7% or more, and still even more preferably 99.9% or more.

According to still another exemplary embodiment of the present invention, an antibacterial strength B measured by the following Method 2 is 55% or more:

[Method 2]

This method is the same as Method 1, except that 0.005 g of the antibacterial composition is added instead of 0.015 g of the antibacterial composition.

According to the exemplary embodiments, excellent antibacterial properties may be provided even when the antibacterial composition is added in an amount of 0.005 g.

According to another exemplary embodiment of the present invention, the antibacterial strength B may be 55% or more, 59% or more, 59.3% or more, 60% or more, and 61.2% or more. In addition, the antibacterial strength B may be 99% or more, preferably 99.3% or more, more preferably 99.5% or more, even more preferably 99.7% or more, and still even more preferably 99.9% or more.

According to still another exemplary embodiment of the present invention, the antibacterial composition according to the above-described exemplary embodiments has an acute oral toxicity dose LD50 of 300 mg/Kg or more, more than 300 mg/Kg, 500 mg/Kg or more, preferably 800 mg/Kg or more, and more preferably 1,000 mg/Kg or more. According to specific examples, the antibacterial composition according to the above-described exemplary embodiments may have an acute oral toxicity dose LD50 of 800 mg/Kg or more, 843 mg/Kg or more, 869 mg/Kg or more, 900 mg/Kg or more, or 998 mg/Kg or more. The antibacterial composition according to the above-described exemplary embodiments may have an acute oral toxicity dose LD50 of 1,000 mg/Kg or more, 1,050 mg/Kg or more, 1,078 mg/Kg or more, 1,100 mg/Kg or more, 1,111 mg/Kg or more, 1,200 mg/Kg or more, 1,242 mg/Kg or more, 1,300 mg/Kg or more, 1,369 mg/Kg or more, 1,400 mg/Kg or more, 1,442 mg/Kg or more, 1,498 mg/Kg or more, 1,500 mg/Kg or more, 1,600 mg/Kg or more, 1,700 mg/Kg or more, and 1,708 mg/Kg or more. According to specific examples, the antibacterial composition according to the above-described exemplary embodiments may have an acute oral toxicity dose LD50 of 843 mg/Kg, 869 mg/Kg, 998 mg/Kg, 1,078 mg/Kg, 1,111 mg/Kg, 1,242 mg/Kg, 1,369 mg/Kg, 1,442 mg/Kg, 1,498 mg/Kg, or 1,708 mg/Kg.

The higher the acute oral toxicity dose LD50 of the antibacterial composition according to the above-described exemplary embodiments, the lower the toxicity, so that the acute oral toxicity dose is advantageous. However, the value of LD50 may be determined from the viewpoint that the above-described antibacterial strength needs also be satisfied. For example, the antibacterial composition according to the above-described exemplary embodiments may have an acute oral toxicity dose LD50 of 50,000 mg/Kg or less, for example, 10,000 mg/Kg or less, 5,000 mg/Kg or less, or 2,000 mg/Kg or less. According to an example, the antibacterial composition according to the above-described exemplary embodiments may have an acute oral toxicity dose LD50 of 1,708 mg/Kg or less.

According to another exemplary embodiment of the present invention, the antibacterial composition according to the above-described exemplary embodiments has an acute percutaneous toxicity dose LD50 of 1,000 mg/Kg or more, more than 1,000 mg/Kg, preferably 1,500 mg/Kg or more, and more preferably 2,000 mg/Kg or more. The higher the acute percutaneous toxicity dose LD50 of the antibacterial composition according to the above-described exemplary embodiments, the lower the toxicity, so that the acute percutaneous toxicity dose is advantageous.

According to an exemplary embodiment of the present invention, the compound comprising a quaternary ammonium structure having an acrylate group or methacrylate group may be selected among those capable of exhibiting the above-described antibacterial strength and acute oral toxicity dose LD50 among the compounds represented by the following Chemical Formula 1:

[Chemical Formula 1]

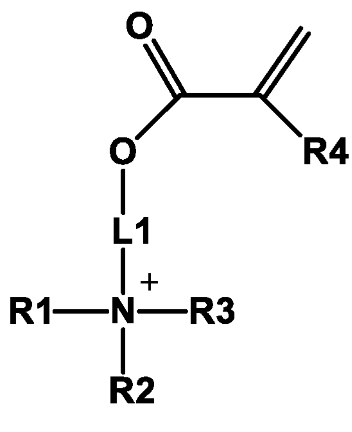

In Chemical Formula 1,

L1 is an alkylene group having 2 to 4 carbon atoms,
R1, R2 and R3 are the same as or different from each other, and are each an alkyl group having 1 to 20 carbon atoms, and
R4 is hydrogen or a methyl group.

According to an exemplary embodiment, at least one of R1, R2 and R3 of Chemical Formula 1 is an alkyl group

having 8 or more carbon atoms, preferably an alkyl group having 8 to 14 carbon atoms, and more preferably an alkyl group having 8 to 12 carbon atoms.

According to an exemplary embodiment, the sum of the number of carbon atoms of the alkyl group comprised in R1, R2 and R3 of Chemical Formula 1 is 12 to 24.

According to an exemplary embodiment, the sum of the number of carbon atoms of two of R1, R2 and R3 having the higher number of carbon atoms in Chemical Formula 1 is 2 to 24, preferably 8 to 20, and more preferably 8 to 16. According to an example, the sum of the number of carbon atoms of two of R1, R2 and R3 having the higher number of carbon atoms in Chemical Formula 1 may be 9 to 15.

According to an exemplary embodiment, the number of carbon atoms of that having the largest number of carbon atoms in R1, R2 and R3 of Chemical Formula 1 is 12 or less, preferably 11 or less.

According to an exemplary embodiment, when any one of two having the largest number of carbon atoms among R1, R2 and R3 of Chemical Formula 1 has 10 or more carbon atoms, the other has less than 8 carbon atoms.

Here, when a group having the largest number of carbon atoms is selected, any one is selected in the case where there are groups having the same carbon atoms.

Acute oral toxicity and acute percutaneous toxicity may be controlled when having the number of carbon atoms according to the aforementioned exemplary embodiments. When acute oral toxicity and/or acute percutaneous toxicity are too high, there are restrictions on intended uses, particularly use in infant products such as diapers.

According to an exemplary embodiment, L1 in Chemical Formula 1 is ethylene or butylene.

According to an exemplary embodiment, R4 in Chemical Formula 1 is hydrogen.

According to an exemplary embodiment, R4 in Chemical Formula 1 is methyl.

The antibacterial composition may be composed of only a compound comprising the quaternary ammonium structure having an acrylate group or methacrylate group, and an additive or solvent may be additionally added, if necessary.

According to another exemplary embodiment of the present invention, the compound comprising the quaternary ammonium structure having the acrylate group or methacrylate group has a water solubility of 50% or more, preferably 60% or more. The solubility may be measured at room temperature.

According to still another exemplary embodiment of the present invention, the compound comprising the quaternary ammonium structure having an acrylate group or methacrylate group has an ethanol solubility of 50% or more, preferably 60% or more. The solubility may be measured at room temperature.

According to yet another exemplary embodiment of the present invention, the compound comprising the quaternary ammonium structure having an acrylate group or methacrylate group may be dissolved in at least one of methanol, acetone, dichloromethane, DMSO, THF, and chloroform, preferably in all of them.

According to yet another exemplary embodiment of the present invention, the compound comprising the quaternary ammonium structure having an acrylate group or methacrylate group may be selected among the following Monomers 1 to 10:

Monomer 1

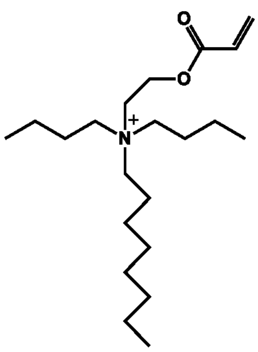

Monomer 2

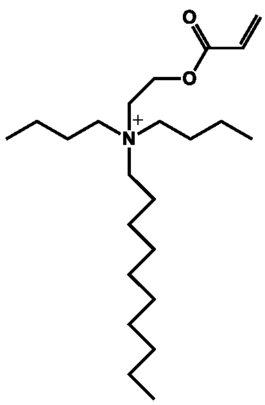

Monomer 3

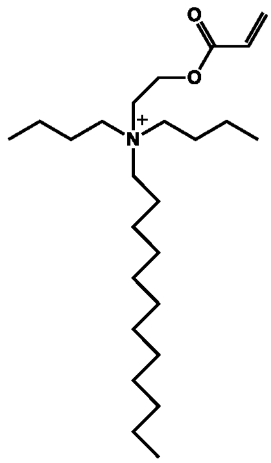

Monomer 4

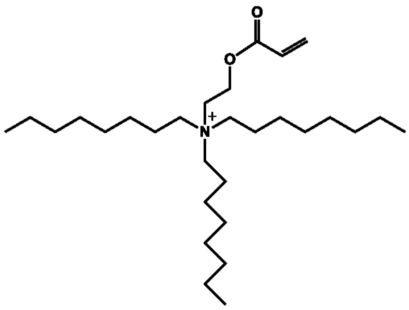

Monomer 5

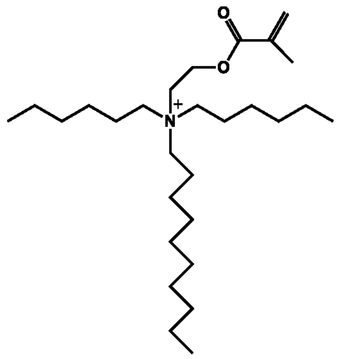

Monomer 6

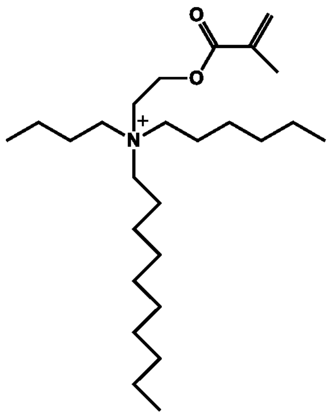

Monomer 7

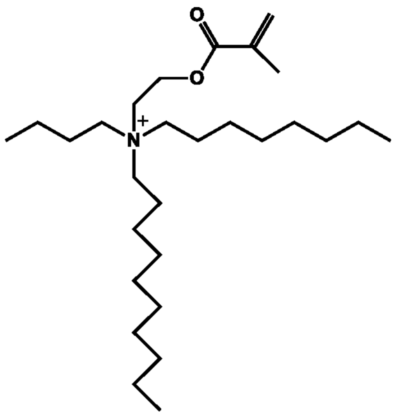

Monomer 8

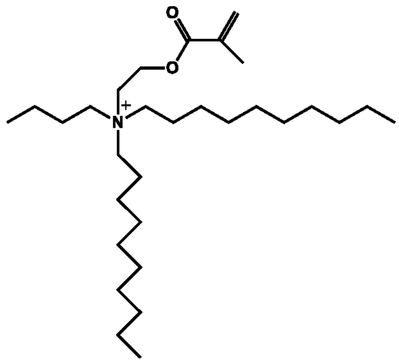

Monomer 9

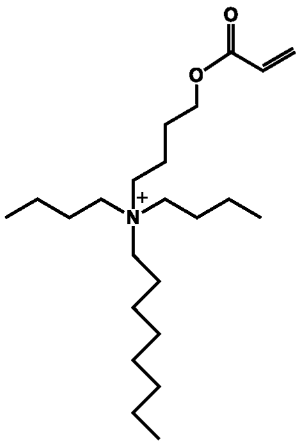

Monomer 10

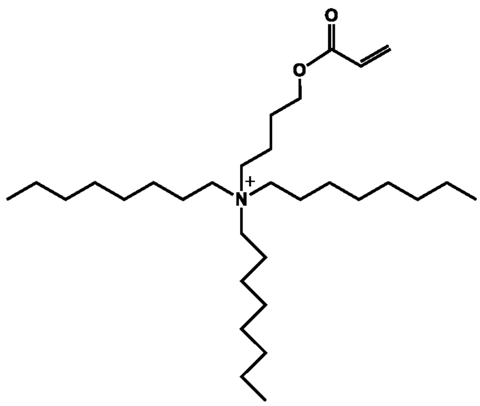

The antibacterial composition according to the above-described exemplary embodiments or the compound comprising the quaternary ammonium structure having an acrylate group or methacrylate group comprised in the same may be present in the form of a powder or oil.

Since the compound comprising the quaternary ammonium structure having an acrylate group or methacrylate

group exhibits cationic properties, the compound may be present in a form where a salt is formed along with a group exhibiting anionic properties. In this case, the group exhibiting anionic properties is not particularly limited, and materials known in the art may be used as long as the materials do not impair the purpose of the antibacterial composition. For example, the group exhibiting anionic properties may be a halogen anion, specifically, $Br^-$.

According to yet another exemplary embodiment of the present invention, provided is a product comprising the antibacterial composition according to the above-described exemplary embodiments or prepared therefrom. The product is not particularly limited as long as antibacterial properties are required. According to an example, the product may be used in a state of a copolymer such as a copolymer with a (meth)acrylate-based resin, a copolymer with polyvinyl chloride, a copolymer with polylactic acid (PLA), and a copolymer with urethane, or may be a product comprising at least one of the copolymers, for example, a hygiene product, an antibacterial film, a diaper, and the like. The copolymer is preferably a copolymer with an acrylate or methacrylate-based compound. As the copolymerization method, a known copolymerization method may be applied.

According to yet another exemplary embodiment of the present invention, provided is a compound selected among the following Monomers 1 to 10. The same description on the above-described compound comprising the quaternary ammonium structure having an acrylate group or methacrylate group may be applied to the following Monomers 1 to 10.

Monomer 1

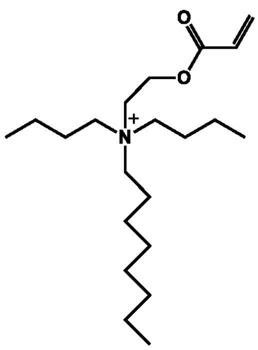

Monomer 2

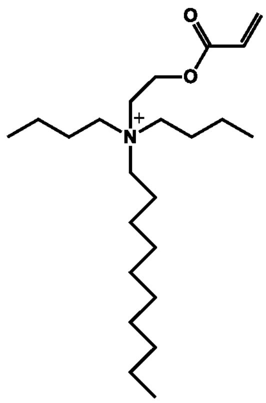

Monomer 3

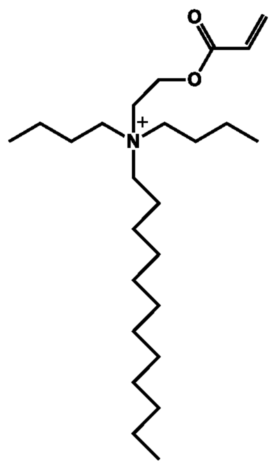

Monomer 4

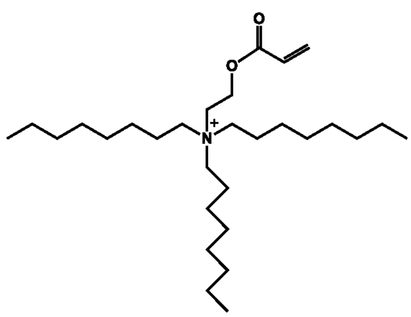

Monomer 5

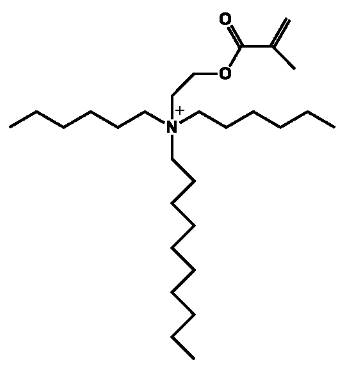

Monomer 6

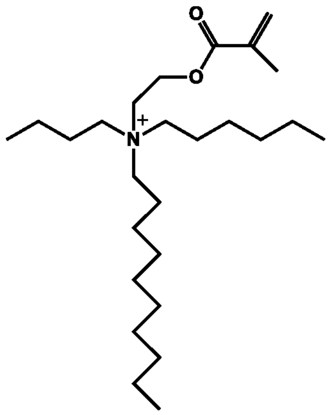

Monomer 7

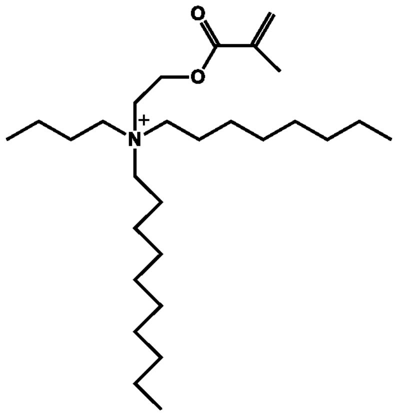

Monomer 8

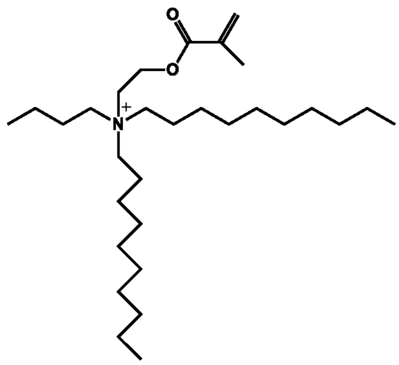

Monomer 9

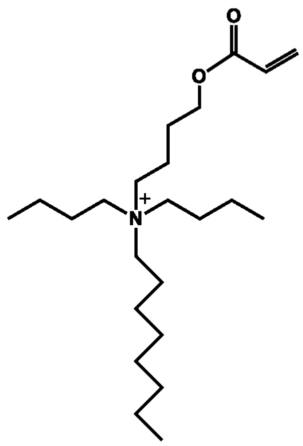

Monomer 10

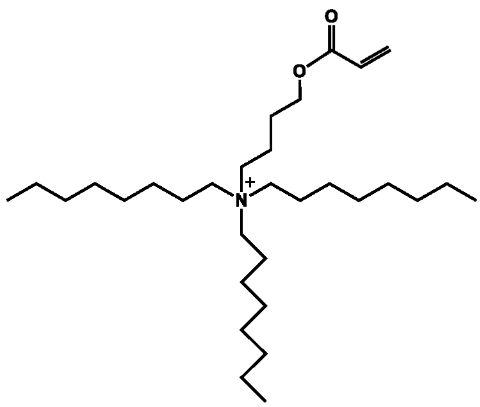

A compound selected among Monomers 1 to 10 may be applied as a constituent component of the antibacterial composition. Furthermore, since the compound selected among Monomers 1 to 10 exhibits cationic properties, the compound may be present in a form where a salt is formed along with a group exhibiting anionic properties. In this case, the group exhibiting anionic properties is not particularly limited, and materials known in the art may be used as long as the materials do not impair the purpose of the antibacterial composition. For example, the group exhibiting anionic properties may be a halogen anion, specifically, $Br^-$.

[Mode for Invention]

Hereinafter, the present invention will be described in more detail through examples. However, the following examples are provided for illustrating the present invention, and the scope of the present invention is not limited thereby.

**[Synthesis of compound comprising quaternary ammonium structure]**

**Synthesis of Monomer 1, Monomer 2, and Monomer 3**

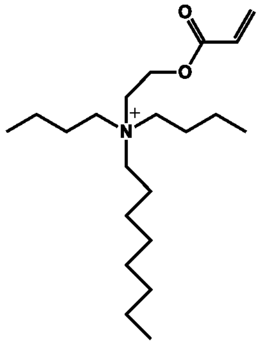 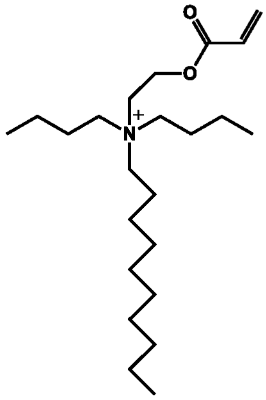 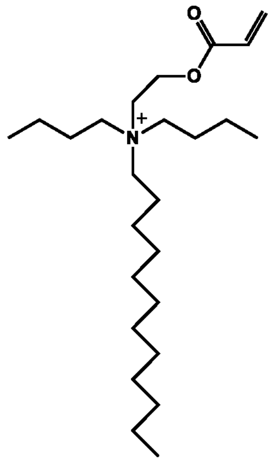

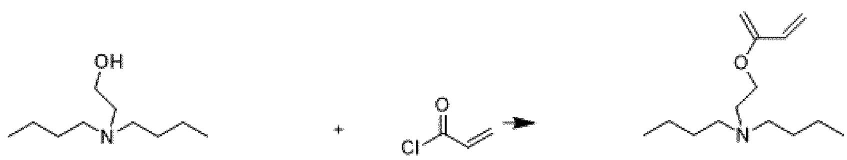

Step 1

1. 0.1 mol of 2-(dibutylamino)ethanol (DBAE), 0.1 mol of trimethylamine and 0.001 mol of hydroquinone were added to 100 mL of THF (solvent).
2. 0.1 mol of acryloyl chloride was added dropwise onto a reaction solution while stirring the materials (room temperature).
3. The resulting mixture was stirred for 2 hours.
4. After a triethylamine salt was removed by filtering the mixture, the solvent was removed by a rotary evaporator.
5. The residue was dried under vacuum at 83 to 87°C.

Step 2

1. The product of Step 1 and 1-bromooctane (preparation of Monomer 1), 1-bromodecane (preparation of Monomer 2), or 1-bromododecane (preparation of Monomer 3) were dissolved at 50 wt% in acrylonitrile (solvent) at a ratio of 1:1.
2. Subsequently, p-methoxyphenol, which is a polymerization inhibitor, was added (ratio with reactants 1:0.001 eq).
3. The resulting mixture was reacted at 50°C for 20 hours.
4. After static precipitation (MTBE:reaction solution = 15:1) in methyl t-butyl ether (MTBE), the mixture was filtered. Here, although a static precipitation method of adding a reactant to a nonsolvent was used, a reverse precipitation method of adding a nonsolvent to a reactant may also be used. Furthermore, in addition to 15:1, other ratios of MTBE and reaction solution may be used, and for example, 12:1 and 26:1 may be used.
5. The resulting product was dried under vacuum at 45°C.

The $^1$H-NMR spectrum ($(CD_3)_2SO$) of the synthesized Monomer 1 is illustrated in the following FIG. 1, the $^1$H-NMR spectrum ($(CD_3)_2SO$) of the synthesized Monomer 2 is illustrated in the following FIG. 2, and the $^1$H-NMR spectrum ($(CD_3)_2SO$) of the synthesized Monomer 3 is illustrated in the following FIG. 3.

**Synthesis of Monomer 4**

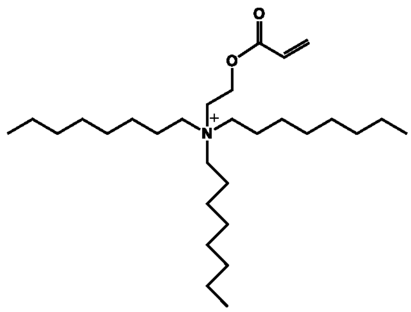

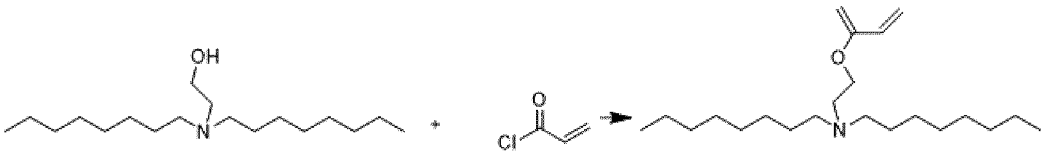

Step 1

1. 0.1 mol of 2-(dioctylamino)ethanol (DOAE), 0.1 mol of trimethylamine and 0.001 mol of hydroquinone were added to 100 mL of THF (solvent).
2. 0.1 mol of acryloyl chloride was added dropwise onto a reaction solution while stirring the materials (room temperature).
3. The resulting mixture was stirred for 2 hours.
4. After a triethylamine salt was removed by filtering the mixture, the solvent was removed by a rotary evaporator.
5. The residue was dried under vacuum at 83 to 87°C.

Step 2

1. The product of Step 1 and 1-bromooctane were dissolved at 50 wt% in acrylonitrile (solvent) at a ratio of 1:1.
2. Subsequently, p-methoxyphenol, which is a polymerization inhibitor, was added (ratio with reactants 1:0.001 eq).
3. The resulting mixture was reacted at 50°C for 20 hours.
4. After static precipitation (MTBE:reaction solution = 15:1) in methyl t-butyl ether (MTBE), the mixture was filtered.
5. The resulting product was dried under vacuum at 45°C.

Synthesis of Monomer 4 was confirmed by $^1$H-NMR spectrum $(CD_3)_2SO$) in a manner similar to the above-described Monomers 1 to 3.

**Synthesis of Monomer 5**

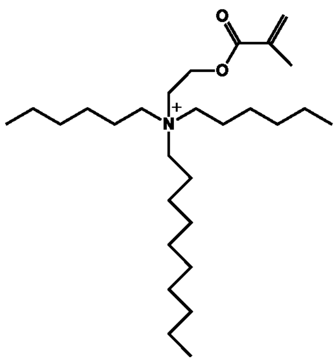

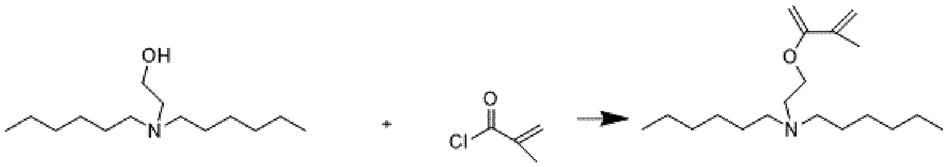

Step 1

1. 0.1 mol of 2-(dihexylamino)ethanol (DHAE), 0.1 mol of trimethylamine and 0.001 mol of hydroquinone were added to 100 mL of THF (solvent).
2. 0.1 mol of methacryloyl chloride was added dropwise onto a reaction solution while stirring the materials (room temperature).
3. The resulting mixture was stirred for 2 hours.
4. After a triethylamine salt was removed by filtering the mixture, the solvent was removed by a rotary evaporator.
5. The residue was dried under vacuum at 83 to 87°C.

Step 2

1. The product of Step 1 and 1-bromodecane were dissolved at 50 wt% in acrylonitrile (solvent) at a ratio of 1:1.
2. Subsequently, p-methoxyphenol, which is a polymerization inhibitor, was added (ratio with reactants 1:0.001 eq).
3. The resulting mixture was reacted at 50°C for 20 hours.
4. After static precipitation (MTBE:reaction solution = 15:1) in methyl t-butyl ether (MTBE), the mixture was filtered.
5. The resulting product was dried under vacuum at 45°C.

Synthesis of monomer 5 was confirmed by $^1$H-NMR spectrum $(CD_3)_2SO$) in a manner similar to the above-described Monomers 1 to 3.

Synthesis of Monomer 6

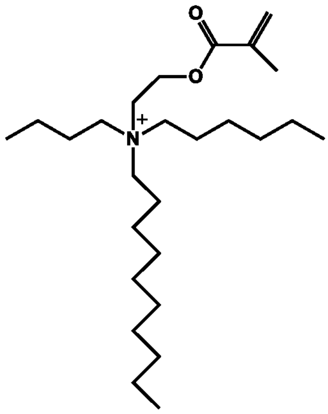

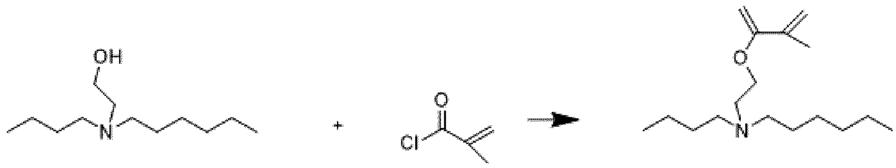

Step 1

1. 0.1 mol of (butylhexylamino)ethanol (BHAE), 0.1 mol of trimethylamine and 0.001 mol of hydroquinone were added to 100 mL of THF (solvent).
2. 0.1 mol of methacryloyl chloride was added dropwise onto a reaction solution while stirring the materials (room temperature).
3. The resulting mixture was stirred for 2 hours.
4. After a triethylamine salt was removed by filtering the mixture, the solvent was removed by a rotary evaporator.
5. The residue was dried under vacuum at 83 to 87°C.

Step 2

1. The product of Step 1 and 1-bromodecane were dissolved at 50 wt% in acrylonitrile (solvent) at a ratio of 1:1.
2. Subsequently, p-methoxyphenol, which is a polymerization inhibitor, was added (ratio with reactants 1:0.001 eq).
3. The resulting mixture was reacted at 50°C for 20 hours.
4. After static precipitation (MTBE:reaction solution = 15:1) in methyl t-butyl ether (MTBE), the mixture was filtered.
5. The resulting product was dried under vacuum at 45°C.

Synthesis of Monomer 6 was confirmed by $^1$H-NMR spectrum ($(CD_3)_2SO$) in a manner similar to the above-described Monomer 1 to 3.

Synthesis of Monomer 7

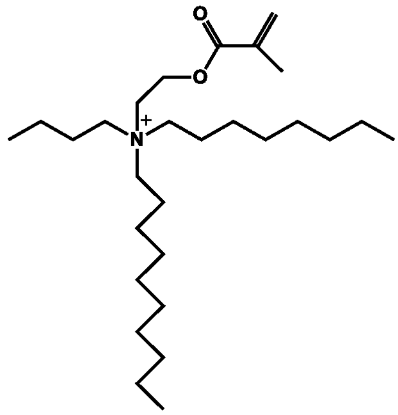

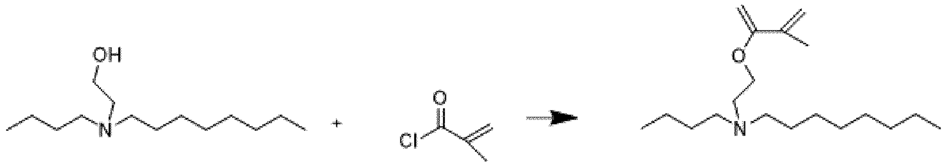

Step 1

1. 0.1 mol of 2-(butyloctylamino)ethanol (BOAE), 0.1 mol of trimethylamine and 0.001 mol of hydroquinone were added to 100 mL of THF (solvent).
2. 0.1 mol of methacryloyl chloride was added dropwise onto a reaction solution while stirring the materials (room temperature).
3. The resulting mixture was stirred for 2 hours.
4. After a triethylamine salt was removed by filtering the mixture, the solvent was removed by a rotary evaporator.
5. The residue was dried under vacuum at 83 to 87°C.

Step 2

1. The product of Step 1 and 1-bromodecane were dissolved at 50 wt% in acrylonitrile (solvent) at a ratio of 1:1.
2. Subsequently, p-methoxyphenol, which is a polymerization inhibitor, was added (ratio with reactants 1:0.001 eq).
3. The resulting mixture was reacted at 50°C for 20 hours.
4. After static precipitation (MTBE:reaction solution = 15:1) in methyl t-butyl ether (MTBE), the mixture was filtered.
5. The resulting product was dried under vacuum at 45°C.

Synthesis of Monomer 7 was confirmed by $^1$H-NMR spectrum ($(CD_3)_2SO$) in a manner similar to the above-described Monomer 1 to 3.

**Synthesis of Monomer 8**

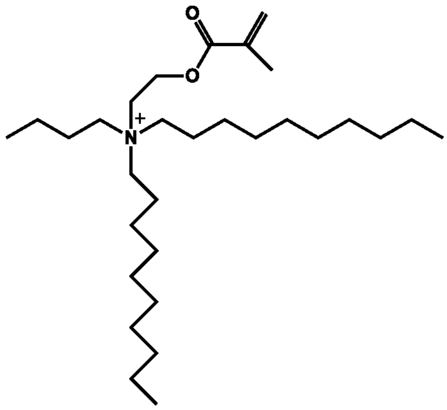

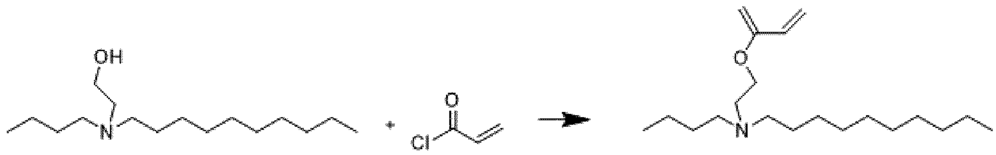

Step 1

1. 0.1 mol of 2-(butyldecylamino)ethanol (BOAE), 0.1 mol of trimethylamine and 0.001 mol of hydroquinone were added to 100 mL of THF (solvent).
2. 0.1mol of methacryloyl chloride was added dropwise onto a reaction solution while stirring the materials (room temperature).
3. The resulting mixture was stirred for 2 hours.
4. After a triethylamine salt was removed by filtering the mixture, the solvent was removed by a rotary evaporator.
5. The residue was dried under vacuum at 83 to 87°C.

Step 2

1. The product of Step 1 and 1-bromodecane were dissolved at 50 wt% in acrylonitrile (solvent) at a ratio of 1:1.
2. Subsequently, p-methoxyphenol, which is a polymerization inhibitor, was added (ratio with reactants 1:0.001 eq).
3. The resulting mixture was reacted at 50°C for 20 hours.
4. After static precipitation (MTBE:reaction solution = 15:1) in methyl t-butyl ether (MTBE), the mixture was filtered.
5. The resulting product was dried under vacuum at 45°C.

Synthesis of Monomer 8 was confirmed by $^1$H-NMR spectrum ($(CD_3)_2SO$) in a manner similar to the above-described Monomer 1 to 3.

**Synthesis of Monomer 9**

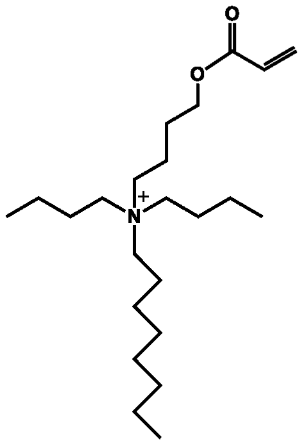

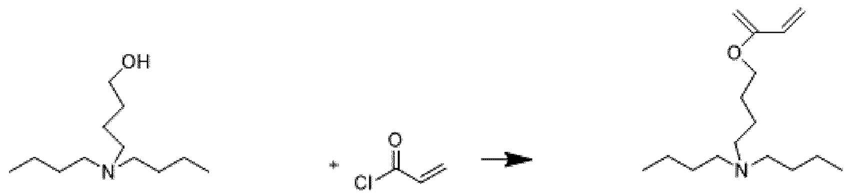

Step 1

1. 0.1 mol of 2-(dibutylamino)butanol (DBAB), 0.1 mol of trimethylamine and 0.001 mol of hydroquinone were added to 100 mL of THF (solvent).
2. 0.1 mol of acryloyl chloride was added dropwise onto a reaction solution while stirring the materials (room temperature).
3. The resulting mixture was stirred for 2 hours.
4. After a triethylamine salt was removed by filtering the mixture, the solvent was removed by a rotary evaporator.
5. The residue was dried under vacuum at 83 to 87°C.

Step 2

1. The product of Step 1 and 1-bromooctane were dissolved at 50 wt% in acrylonitrile (solvent) at a ratio of 1:1.
2. Subsequently, p-methoxyphenol, which is a polymerization inhibitor, was added (ratio with reactants 1:0.001 eq).
3. The resulting mixture was reacted at 50°C for 20 hours.
4. After static precipitation (MTBE:reaction solution = 15:1) in methyl t-butyl ether (MTBE), the mixture was filtered.
5. The resulting product was dried under vacuum at 45°C.

Synthesis of Monomer 9 was confirmed by $^1$H-NMR spectrum $(CD_3)_2SO$) in a manner similar to the above-described Monomers 1 to 3.

Synthesis of Monomer 10

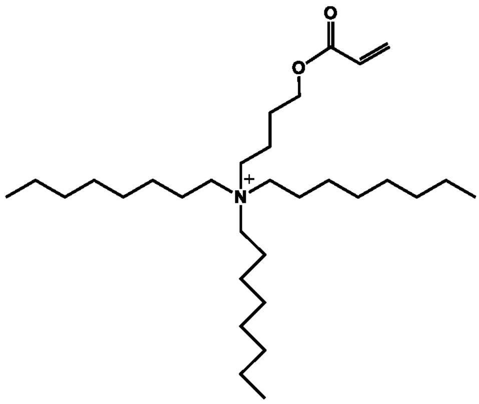

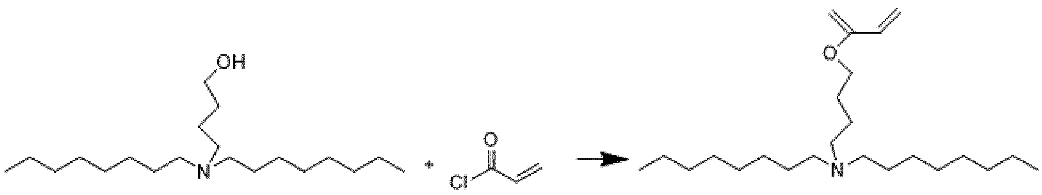

Step 1

1. 0.1 mol 2-(dioctylamino)butanol (DOAB), 0.1 mol of trimethylamine and 0.001 mol of hydroquinone were added to 100 mL of THF (solvent).
2. 0.1 mol of acryloyl chloride was added dropwise onto a reaction solution while stirring the materials (room temperature).
3. The resulting mixture was stirred for 2 hours.
4. After a triethylamine salt was removed by filtering the mixture, the solvent was removed by a rotary evaporator.
5. The residue was dried under vacuum at 83 to 87°C.

Step 2

1. The product of Step 1 and 1-bromooctane were dissolved at 50 wt% in acrylonitrile (solvent) at a ratio of 1:1.
2. Subsequently, p-methoxyphenol, which is a polymerization inhibitor, was added (ratio with reactants 1:0.001 eq).
3. The resulting mixture was reacted at 50°C for 20 hours.
4. After static precipitation (MTBE:reaction solution = 15:1) in methyl t-butyl ether (MTBE), the mixture was filtered.
5. The resulting product was dried under vacuum at 45°C.

Synthesis of Monomer 10 was confirmed by $^1$H-NMR spectrum ($(CD_3)_2SO$) in a manner similar to the above-described Monomer 1 to 3.

**Synthesis of Comparative Monomer 1**

Synthesis was performed in the same manner as in the synthesis process of Monomer 1, except that bromohexane was used instead of 1-bromooctane of Step 2 in the synthesis process of Monomer 1.

Synthesis of Comparative Monomer 1 was confirmed by $^1$H-NMR spectrum ($(CD_3)_2SO$) in a manner similar to the above-described Monomers 1 to 3.

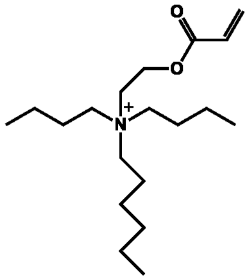

**Synthesis of Comparative Monomer** 2

Synthesis was performed in the same manner as in the synthesis process of Monomer 10, except that 2-(ditetradecylamino)butanol was used instead of 2-(dioctylamino)butanol of Step 1 and bromotetradecane was used instead of 1-bromooctane of Step 2 in the synthesis process of Monomer 10.

Synthesis of Comparative Monomer 2 was confirmed by $^1$H-NMR spectrum ($(CD_3)_2SO$) in a manner similar to the above-described Monomers 1 to 3.

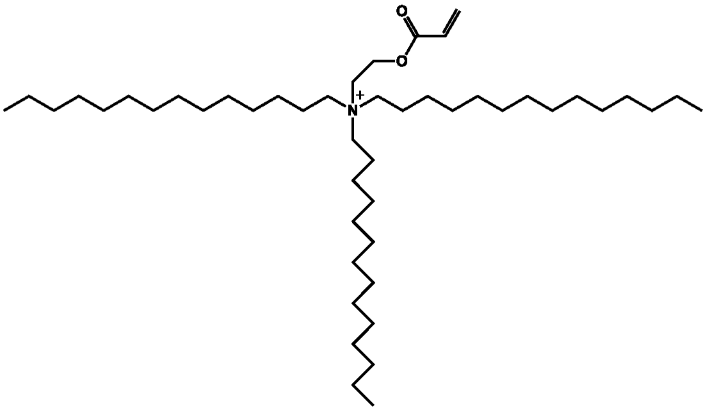

The antibacterial strength of Monomers 1 to 10 and Comparative Monomers 1 and 2 by Methods 1 to 4 was measured, and is shown in the following Table 1. In this case, the *P.mirabilis* (ATCC29906) bacteria were used.

[Table 1]

| No. | Sample | Antibacterial strength (%) | | | |
|---|---|---|---|---|---|
| | | Method 2 (amount of antibacterial composition added 0.005g) | Method 4 (amount of antibacterial composition added 0.01g) | Method 1 (amount of antibacterial composition added 0.015g) | Method 3 (amount of antibacterial composition added 0.02g) |
| 1 | reference | - | - | - | - |
| 2 | Monomer 1 | 59.3 | 68.2 | 99.9 | 99.9 |
| 3 | Monomer 2 | 99.9 | 99.9 | 99.9 | 99.9 |
| 4 | Monomer 3 | 99.9 | 99.9 | 99.9 | 99..9 |
| 5 | Monomer 4 | 99.9 | 99.9 | 99.9 | 99.9 |
| 6 | Monomer 5 | 99.9 | 99.9 | 99.9 | 99.9 |
| 7 | Monomer 6 | 99.9 | 99.9 | 99.9 | 99.9 |
| 8 | Monomer 7 | 99.9 | 99.9 | 99.9 | 99.9 |
| 9 | Monomer 8 | 99.9 | 99.9 | 99.9 | 99.9 |
| 10 | Monomer 9 | 61.2 | 70.0 | 99.9 | 99.9 |

(continued)

| No. | Sample | Antibacterial strength (%) | | | |
|---|---|---|---|---|---|
| | | Method 2 (amount of antibacterial composition added 0.005g) | Method 4 (amount of antibacterial composition added 0.01g) | Method 1 (amount of antibacterial composition added 0.015g) | Method 3 (amount of antibacterial composition added 0.02g) |
| 11 | Monomer 10 | 99.9 | 99.9 | 99.9 | 99.9 |
| 12 | Comparative Monomer 1 | 22.6 | 30.7 | 38.8 | 46.9 |
| 13 | Comparative Monomer 2 | 99.9 | 99.9 | 99.9 | 99.9 |

According to Table 1, all Monomers 1 to 10 had an antibacterial strength A of 99.9% measured by Method 1.

The time at which 99.9% of the antibacterial strength of Monomers 1 to 10 and Comparative Monomers 1 and 2 is confirmed is shown in the following Table 2.

[Table 2]

| No. | Sample | Antibacterial strength (%) | | | |
|---|---|---|---|---|---|
| | | Method 2 (amount of antibacterial composition added 0.005g) | Method 4 (amount of antibacterial composition added 0.01g) | Method 1 (amount of antibacterial composition added 0.015g) | Method 3 (amount of antibacterial composition added 0.02g) |
| 1 | reference | - | - | - | - |
| 2 | Monomer 1 | - | - | Within 1 hour | Within 1 hour |
| 3 | Monomer 2 | Within 1 hour | Within 1 hour | Within 1 hour | Within 1 hour |
| 4 | Monomer 3 | Within 1 hour | Within 1 hour | Within 1 hour | Within 1 hour |
| 5 | Monomer 4 | Within 1 hour | Within 1 hour | Within 1 hour | Within 1 hour |
| 6 | Monomer 5 | Within 1 hour | Within 1 hour | Within 1 hour | Within 1 hour |
| 7 | Monomer 6 | Within 1 hour | Within 1 hour | Within 1 hour | Within 1 hour |
| 8 | Monomer 7 | Within 1 hour | Within 1 hour | Within 1 hour | Within 1 hour |
| 9 | Monomer 8 | Within 1 hour | Within 1 hour | Within 1 hour | Within 1 hour |
| 10 | Monomer 9 | - | - | Within 1 hour | Within 1 hour |
| 11 | Monomer 10 | Within 1 hour | Within 1 hour | Within 1 hour | Within 1 hour |
| 12 | Comparative Monomer 1 | - | - | - | - |
| 13 | Comparative Monomer 2 | Within 1 hour | Within 1 hour | Within 1 hour | Within 1 hour |

According to Table 2, all Monomers 1 to 10 having an antibacterial strength A of 99.9% measured by Method 1 was confirmed within 1 hour.

The acute oral toxicity dose LD50 of Monomers 1 to 10 and Comparative Monomers 1 and 2 is shown in the following Table 3.

[Table 3]

| Sample | Acute oral toxicity dose (mg/Kg) LD50 |
|---|---|
| Monomer 1 | 1,708 |

(continued)

| Sample | Acute oral toxicity dose (mg/Kg) LD50 |
|---|---|
| Monomer 2 | 1,369 |
| Monomer 3 | 843 |
| Monomer 4 | 1,442 |
| Monomer 5 | 1,078 |
| Monomer 6 | 1,111 |
| Monomer 7 | 998 |
| Monomer 8 | 869 |
| Monomer 9 | 1,498 |
| Monomer 10 | 1,242 |
| Comparative Monomer 1 | 2,355 |
| Comparative Monomer 2 | 231 |

According to Table 3, all Monomers 1 to 10 had an acute oral toxicity dose LD50 of 300 mg/Kg or more, particularly 800 mg/Kg or more. Furthermore, Examples 1, 2, 4, 5, 6, 9 and 10 had an acute oral toxicity dose LD50 of 1,000 mg/Kg or more.

The acute percutaneous toxicity dose LD50 of Monomers 1 to 10 is shown in the following Table 4.

[Table 4]

| Sample | Acute percutaneous toxicity dose (mg/Kg) LD50 |
|---|---|
| Monomer 1 | 2,000 or more |
| Monomer 2 | 2,000 or more |
| Monomer 3 | 2,000 or more |
| Monomer 4 | 2,000 or more |
| Monomer 5 | 2,000 or more |
| Monomer 6 | 2,000 or more |
| Monomer 7 | 2,000 or more |
| Monomer 8 | 2,000 or more |
| Monomer 9 | 2,000 or more |
| Monomer 10 | 2,000 or more |

According to Table 4, all Monomers 1 to 10 had an acute percutaneous toxicity dose LD50 of 1,000 mg/Kg or more, particularly 2,000 mg/Kg or more.

The acute oral toxicity dose LD50 and acute percutaneous toxicity dose LD50 were measured by 3T3 Neutral Red Uptake (NRU) assay (OECD Guidance Document No 129). Specifically, the acute oral toxicity dose LD50 and acute percutaneous toxicity dose LD50 may be calculated by the following method.

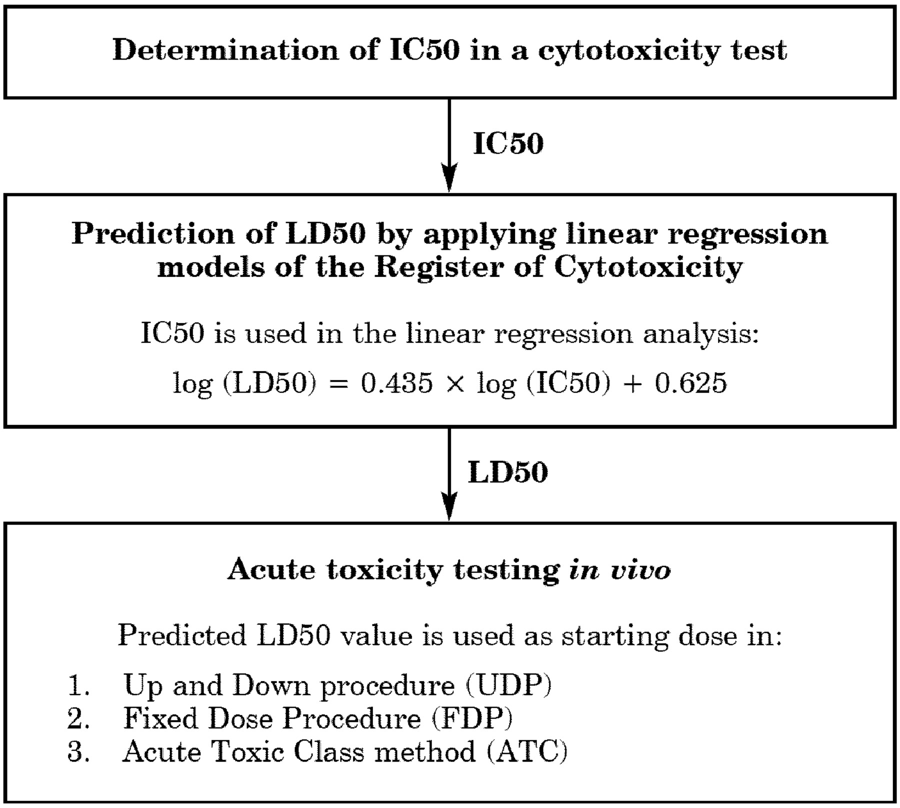


## Claims

1. An antibacterial composition comprising at least one compound comprising a quaternary ammonium structure having an acrylate group or methacrylate group, and having an antibacterial strength A of 90% or more which is measured by the following Method 1 for at least one strain of Gram-positive bacteria and Gram-negative bacteria and an acute oral toxicity dose LD50 of 300 mg/Kg or more:

[Method 1]
After 25 ml of a broth-type medium (Nutrient broth, BD DIFCO., 8 g/L) inoculated with 3,000 CFU/ml bacteria is put into a 50 mL conical tube, 0.015 g of the antibacterial composition is added thereto and suspended (vortexing), a sufficiently mixed solution is incubated for 16 hours in a shaking water bath maintained at 35°C. After the incubated solution was diluted to 1/5 using a 1X PBS buffer solution, absorbance (λ = 600 nm) is measured using a UV/Vis spectrophotometer, and the antibacterial strength, which is a bacteriostatic reduction rate, is calculated by the following equation by comparing the measured absorbance with a solution incubated without the addition of the antibacterial composition.

$$\text{Antibacterial strength (\%)} = \left(1 - {A_{sample}}/{A_{Reference}}\right) \times 100$$

$A_{sample}$ = Absorbance of medium solution incubated by adding antibacterial composition

$A_{reference}$ = Absorbance of medium solution incubated without addition of antibacterial composition

**2.** The antibacterial composition of claim 1, wherein Method 1 is measured against at least one strain of *Proteus mirabilis, E.coli, S.aureus*, *E.Cloaceae* and *E.faecalis*.

**3.** The antibacterial composition of claim 1, wherein Method 1 is measured for each strain of *Proteus mirabilis, E. coli, S. aureus, E. Cloaceae* and *E.faecalis*.

**4.** The antibacterial composition of any one of claims 1 to 3, wherein an antibacterial strength A of the antibacterial composition is expressed within 1 hour.

**5.** The antibacterial composition of any one of claims 1 to 3, wherein a ratio (CB) of an antibacterial strength C measured by the following Method 3 to an antibacterial strength B measured by the following Method 2 is 1 or more and less than 2:

[Method 2]
This method is the same as Method 1, except that 0.005 g of the antibacterial composition is added instead of 0.015 g of the antibacterial composition.
[Method 3]
This method is the same as Method 1, except that 0.02 g of the antibacterial composition is added instead of 0.015 g of the antibacterial composition.

**6.** The antibacterial composition of any one of claims 1 to 3, wherein the antibacterial strength A is 99% or more.

**7.** The antibacterial composition of any one of claims 1 to 3, wherein an antibacterial strength D measured by the following Method 4 is 65% or more:

[Method 4]
This method is the same as Method 1, except that 0.01 g of the antibacterial composition is added instead of 0.015 g of the antibacterial composition.

**8.** The antibacterial composition of claim 7, wherein the antibacterial strength D is 99% or more.

**9.** The antibacterial composition of any one of claims 1 to 3, wherein the antibacterial strength B measured by the following Method 2 is 55% or more:

[Method 2]
This method is the same as Method 1, except that 0.005 g of the antibacterial composition is added instead of 0.015 g of the antibacterial composition.

**10.** The antibacterial composition of claim 9, wherein the antibacterial strength B is 99% or more.

**11.** The antibacterial composition of any one of claims 1 to 3, wherein the acute oral toxicity dose LD50 is 800 mg/Kg or more.

**12.** The antibacterial composition of any one of claims 1 to 3, wherein the acute oral toxicity dose LD50 is 1,000 mg/Kg or more.

**13.** The antibacterial composition of any one of claims 1 to 3, wherein the compound comprising the quaternary ammonium structure having an acrylate group or methacrylate group is selected among compounds represented by the following Chemical Formula 1:

[Chemical Formula 1]

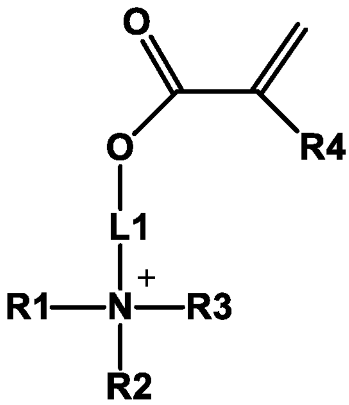

in Chemical Formula 1,

L1 is an alkylene group having 2 to 4 carbon atoms,
R1, R2 and R3 are the same as or different from each other, and are each an alkyl group having 1 to 20 carbon atoms, and
R4 is hydrogen or a methyl group.

**14.** The antibacterial composition of claim 13, wherein at least one of R1, R2 and R3 of Chemical Formula 1 is an alkyl group having 8 or more carbon atoms, and the sum of the number of carbon atoms of the alkyl group comprised in R1, R2 and R3 is 12 to 24.

**15.** The antibacterial composition of any one of claims 1 to 3, wherein the compound comprising the quaternary ammonium structure having an acrylate group or methacrylate group is selected among the following Monomers 1 to 10:

Monomer 1

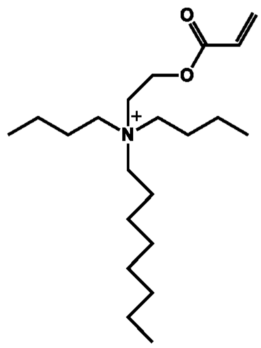

Monomer 2

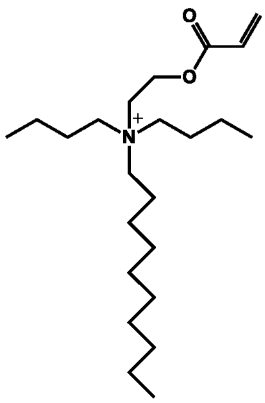

Monomer 3

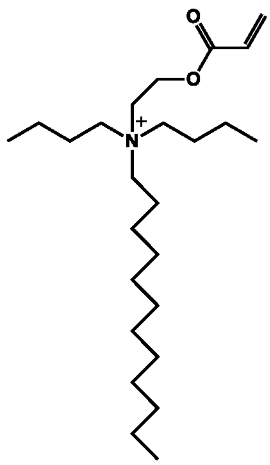

Monomer 4

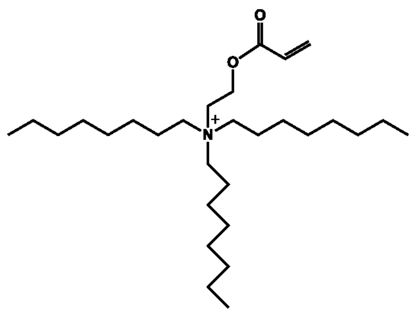

Monomer 5

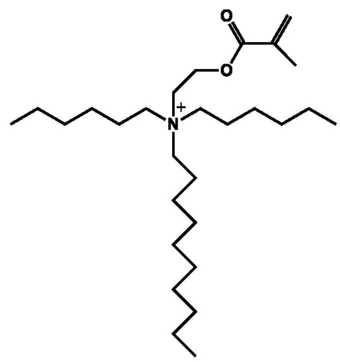

Monomer 6

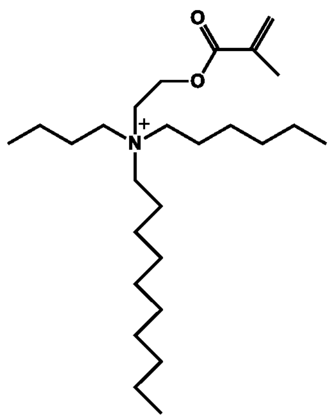

Monomer 7

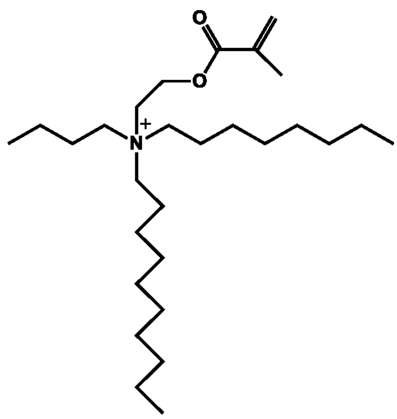

Monomer 8

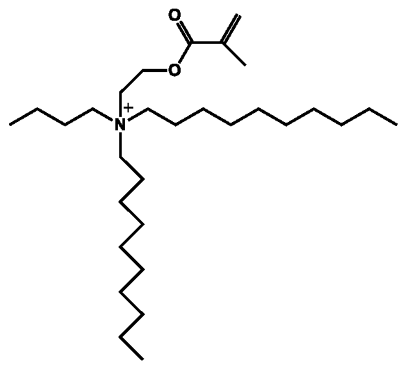

Monomer 9

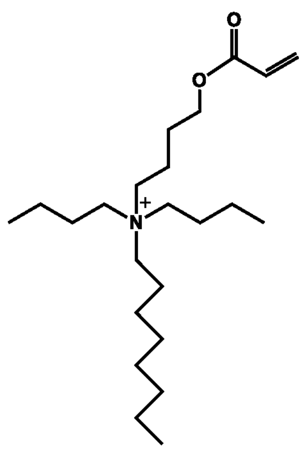

Monomer 10

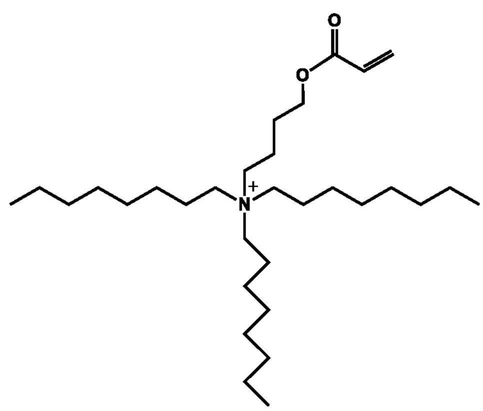

**16.** The antibacterial composition of any one of claims 1 to 3, wherein the antibacterial composition has an acute percutaneous toxicity dose LD50 of 1,000 mg/Kg or more.

**17.** The antibacterial composition of any one of claims 1 to 3, wherein the antibacterial composition has an acute

percutaneous toxicity dose LD50 of 2,000 mg/Kg or more.

**18.** A product comprising the antibacterial composition of any one of claims 1 to 3, or prepared therefrom.

**19.** A compound selected among the following Monomers 1 to 10:

Monomer 1

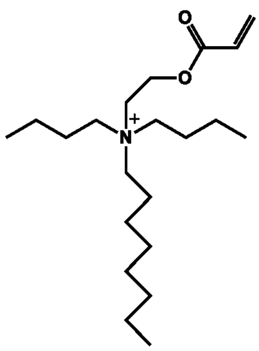

Monomer 2

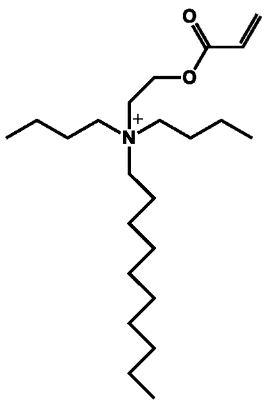

Monomer 3

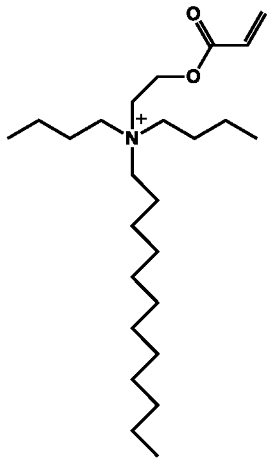

Monomer 4

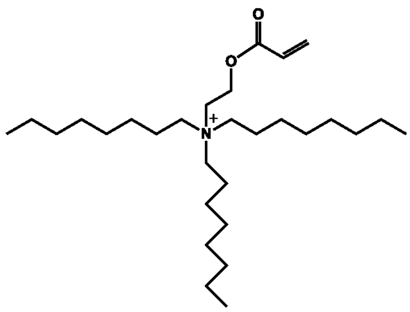

Monomer 5

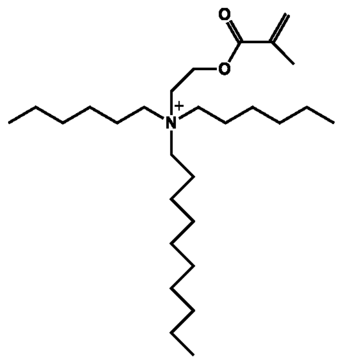

Monomer 6

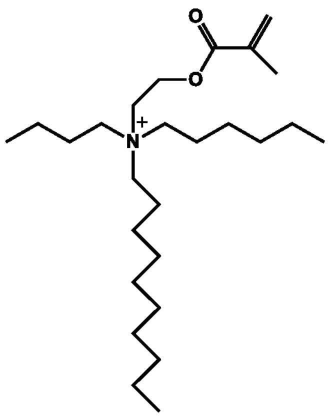

Monomer 7

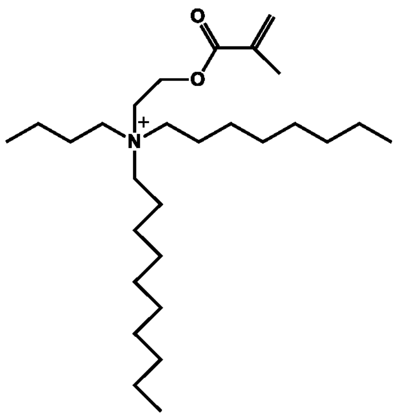

Monomer 8

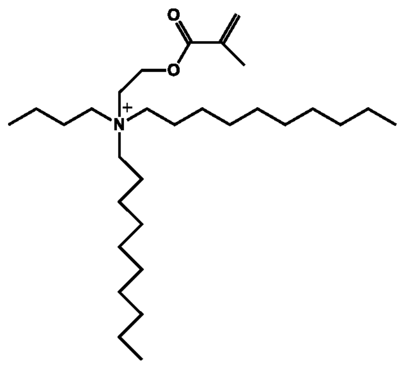

Monomer 9

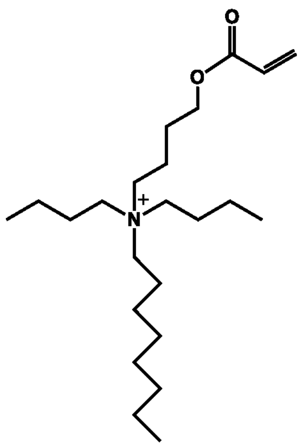

Monomer 10

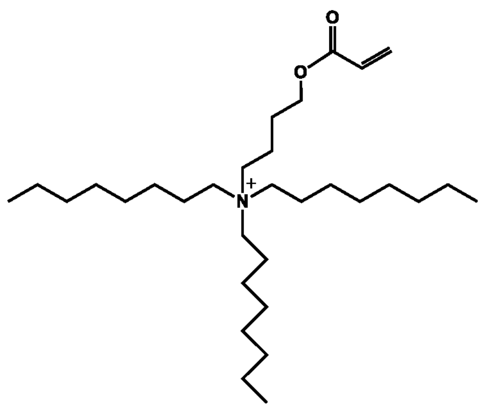

[Figure 1]

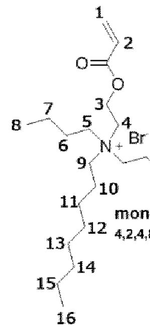

monomer 1
4,2,4,8

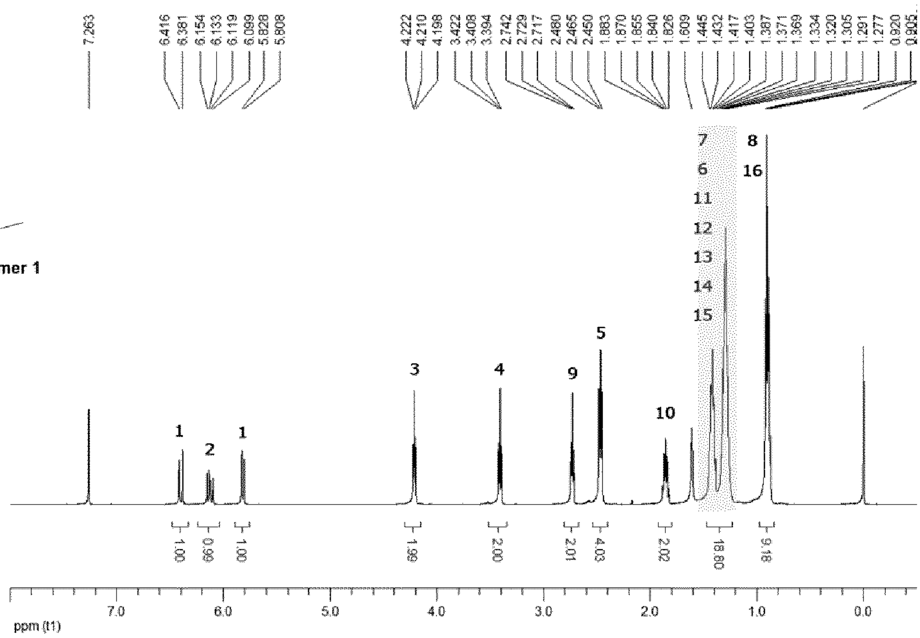


[Figure 2]

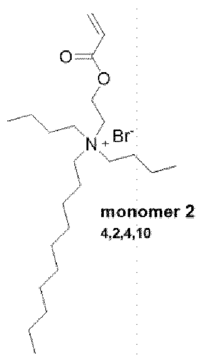

monomer 2
4,2,4,10

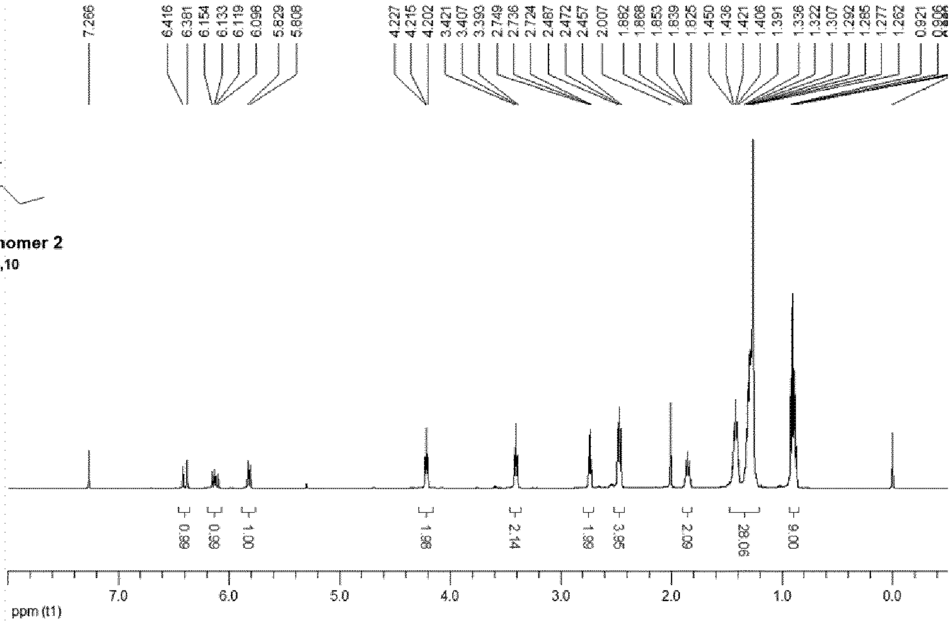

[Figure 3]

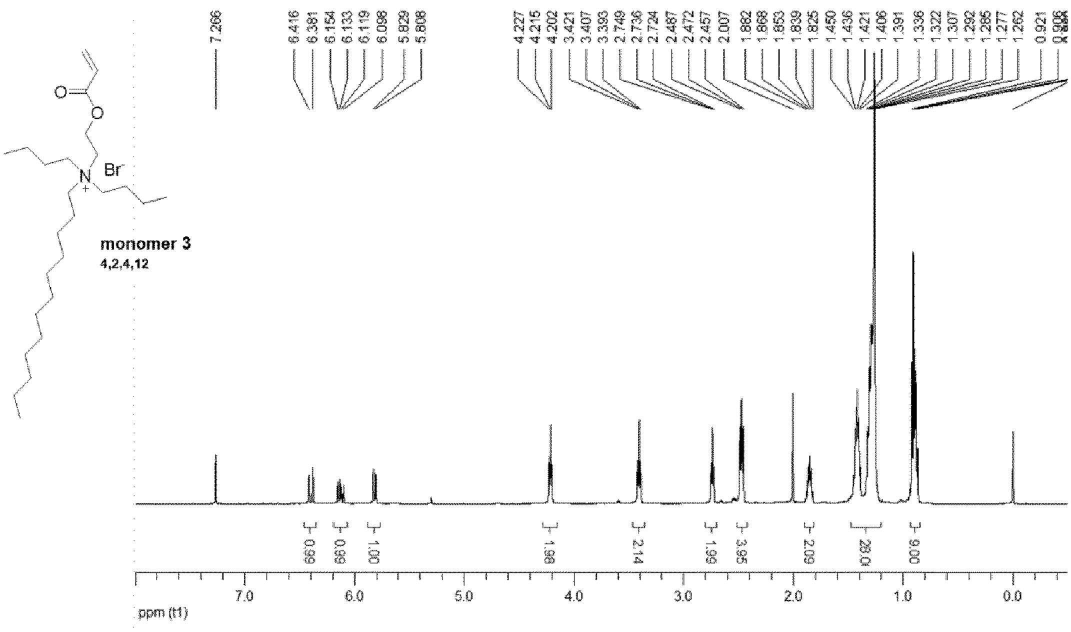
monomer 3
4,2,4,12
7.266
6.416
6.381
6.154
6.133
6.119
6.098
5.829
5.808
4.227
4.215
4.202
3.421
3.407
3.393
2.749
2.736
2.724
2.487
2.472
2.457
2.007
1.882
1.868
1.853
1.839
1.825
1.450
1.436
1.421
1.406
1.391
1.336
1.322
1.307
1.292
1.285
1.277
1.262
0.921
0.99
0.99
1.00
1.98
2.14
1.99
3.95
2.09
28.0
9.00
7.0
6.0
5.0
4.0
3.0
2.0
1.0
0.0
ppm (t1)

## INTERNATIONAL SEARCH REPORT

International application No.
**PCT/KR2022/009358**

**A. CLASSIFICATION OF SUBJECT MATTER**

**A01N 33/12**(2006.01)i

According to International Patent Classification (IPC) or to both national classification and IPC

**B. FIELDS SEARCHED**

Minimum documentation searched (classification system followed by classification symbols)

A01N 33/12(2006.01); A01N 25/04(2006.01); G02B 1/10(2006.01); G02B 5/26(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (registry, caplus), Google & keywords: 항균(antibacterial), 친수성(hydrophilic), 소수성(hydrophobic), 아크릴레이트(acrylate), 메타크릴레이트(methacrylate), 4급 암모늄(quaternary ammonium), 그람 양성균(gram-positive bacteria), 그람 음성균(gram-negative bacteria)

**C. DOCUMENTS CONSIDERED TO BE RELEVANT**

| Category* | Citation of document, with indication, where appropriate, of the relevant passages | Relevant to claim No. |
|---|---|---|
| X | MOHAMMED, M. et al. Antibacterial activity of quaternary ammonium salt from diethylaminoethyl methacrylate. E-Journal of Chemistry. 2010, vol. 7, no. S1, pp. S61-S66.<br>See abstract; pages S62 and S65; formulas 1-3; and table 1. | 1-19 |
| X | DAOUDI, S. et al. Synthesis, Characterizationand Antibacterial Activity of Quaternary Ammonium Compounds Bearing Mixed N-Substituted Groups. Chem. Sci. Trans. 2014, vol. 3, no. 1, pp. 281-291.<br>See abstract; pages 284 and 289-290; figure 1; and table 5. | 1-19 |
| X | MANCUSO, R. et al. Synthesis and antibacterial activity of polymerizable acryloyloxyalkyltriethyl ammonium salts. ChemPlusChem. 2017, vol. 82, no. 10, pp. 1235-1244.<br>See abstract; Introduction and Antimicrobial tests; formulas 1 and 2; and page 1236, right column, lines 8-13. | 1-19 |
| A | JP 2012-208169 A (KONICA MINOLTA HOLDINGS INC.) 25 October 2012 (2012-10-25)<br>See entire document. | 1-19 |
| A | JP 2000-154105 A (DAICEL CHEM. IND. LTD.) 06 June 2000 (2000-06-06)<br>See entire document. | 1-19 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 October 2022** | **12 October 2022** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

**INTERNATIONAL SEARCH REPORT**
**Information on patent family members**

International application No.
**PCT/KR2022/009358**

| Patent document cited in search report | Publication date (day/month/year) | Patent family member(s) | Publication date (day/month/year) |
|---|---|---|---|
| JP 2012-208169 A | 25 October 2012 | JP 5699738 B2 | 15 April 2015 |
| JP 2000-154105 A | 06 June 2000 | None | |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020210093569